# EUROPEAN PATENT APPLICATION

(11) **EP 4 056 167 A1**
(43) Date of publication of application: **14.09.2022**
(21) Application number: 21162346.7
(22) Date of filing: 12.03.2021
(51) Int. Cl.: A61K 9/02, A61K 9/00, A61K 47/44, A61P 1/00, A61P 1/12, A61K 9/19

(54) **MODIFICATION OF FECAL MICROBIOTA WITH RECTAL SUPPOSITORIES**

(71) Applicant: Lietuvos Sveikatos Mokslu Universitetas, 44307 Kaunas (LT)
(72) Inventor: Kup inskas, Juozas, 44307 Kaunas (LT); Skiecevi ien , Jurgita, 44307 Kaunas (LT); Gedgaudas, Rolandas, 44307 Kaunas (LT); Kiudelis, Vytautas, 44307 Kaunas (LT); Bernatonien , Jurga, 44307 Kaunas (LT); Ar tikyt , Justina, 44307 Kaunas (LT)
(74) Representative: Zaboliene, Reda

(57) **Abstract**

We provide a method of preparing a rectal suppository containing lyophilized donor fecal matter. The suppository may be used to administer microbiota directly into the large intestine of a patient for the treatment of various diseases or pathological conditions, where such an intervention is indicated. We also provide results of bacterial composition analysis that was performed on these suppositories. The cocoa butter and Witepsol base of the suppositories as well as the storage conditions of the suppositories did not influence the bacterial composition, richness, and alpha diversity of the lyophilized fecal matter in the suppositories and was like the FMT suspension of the donor.

## Description

### FIELD OF THE INVENTION

The present invention relates to the fecal microbiota transplantation. We have developed suppositories for rectal administration, containing fecal microbiota. The present invention relates to method of producing a rectal suppository containing lyophilized donor fecal matter.

### DESCRIPTION OF THE RELATED ART

Fecal microbiota transplantation is currently the most effective treatment for recurrent *Clostridioides* (formerly *Clostridium*) *difficile* infection. Furthermore, this treatment method is being widely investigated for use in other diseases (e.g., Ulcerative colitis), therefore, it is highly likely that there will be an increase in demand for fecal microbiota modification in the future.

The most commonly used methods for fecal microbiota delivery are direct application via endoscope (during colonoscopy) or administration via nasojejunal or nasoduodenal (enteric) tube. The main downsides of these methods are the possibility of complications, the need for specialized personnel, and the discomfort caused to the patient. A non-invasive method of fecal microbiota transplantation using oral capsules is available, however, there is evidence that it is less effective(1) and the repeat of procedures may be necessary.

AU20190219826 relates to formulations encompassing human fecal extract and the use of such formulations to modify or enhance the microbiota of the human large intestine, or to treat various diseases and/or pathological conditions.

US2018110810 relates to lyophilized formulations, including human fecal extract and a cryoprotectant, and the use of such compositions to modify or enhance the microbiota of the human large intestine, or to treat various diseases and/or pathological conditions.

EP20170188258 relates to a hard capsule, consisting of an outer layer containing a hydrophobic fluid, and inner layer, containing microorganisms and single chosen desiccant. The document also mentions methods of treating human dysbiosis using at least one of the presented capsules.

CN20191047067 reveals a method of producing a capsule containing highly active fecal microbiota. The described production method ensured that the intestinal flora retains its activity and can be stored in low temperatures for an extended period of time. This method is convenient as it does not require an invasive procedure to facilitate fecal microbiota delivery.

Using a suppository as a means of microbiota delivery would result in a quick, safe and inexpensive way to administer fecal microbiota preparations directly into the human large intestine and would result in a higher availability of this treatment method. The advantage of using suppositories as opposed to administering microbiota during colonoscopy or via enteric tube, is that neither specialized personnel nor equipment is required, and it results in lower costs. Furthermore, there would be less complications and patient discomfort related to invasive procedures. When compared to oral capsules, suppositories have the advantage of delivering the formulation directly into the large intestine, whereas capsules need to be resistant to the acidic environment of the stomach, therefore slow-dissolving, double-layered capsules are being used and this results in variable availability of capsule's content in the large intestines of different individuals.

Formulations containing bacteria have been administered by way of suppositories before, to treat conditions related to dysbiosis of the female genital tract e.g., cystitis (2).

### SUMMARY OF THE INVENTION

This invention describes a rectal suppository, consisting of base component and lyophilized donor fecal matter which comprises donor fecal microbiota. The suppository facilitates the administration of the donor fecal matter directly into the large intestine, thus avoiding the transit through the entire digestive tract and eliminating the need of invasive procedures.

In one embodiment the rectal suppository consists of cocoa butter and lyophilized donor fecal matter.

In another embodiment the rectal suppository consists of Witepsol and lyophilized donor fecal matter.

A suppository is prepared for rectal administration.

The suppository composition is prepared by a method which comprises:
a) melting of base component;
b) grinding lyophilized donor fecal matter into fine powder;
c) mixing lyophilized donor fecal matter with cocoa butter;
d) forming suppositories and left until suppositories fully form.

According to one of embodiments a cocoa better used in step a) is melted at 34-37°C.

According to one of embodiments a Witepsol used in step a) is melted at 60-70°C and is cooled to 47-53°C before the mixing with fecal matter. A fecal matter is lyophilized before step b). The resulting mass is constantly mixed when it cools to avoid separation of base element and lyophilized donor fecal matter.

Prepared suppository composition is intended to use in the treatment of disease or pathological condition of the colon.

The suppository composition is intended for use in the treatment of Clostridioides difficile colitis, antibiotic associated diarrhea, irritable bowel syndrome, functional constipation and inflammatory bowel diseases (ulcerative colitis and Crohn's disease).

### BRIEF DESCRIPTION OF DRAWINGS

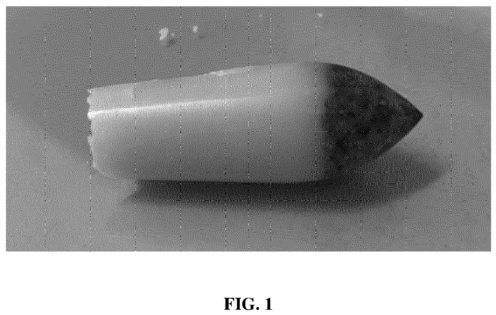

Fig. 1 shows image of a prepared suppository.
Fig. 2 shows donor fecal, FMT suspension, lyophilized fecal matter and suppositories containing lyophilized donor's fecal filtrate microbiota composition comparison. A - multidimensional scaling (MDS) plot. **B-D** - comparison of the bacterial alpha diversity (Simpson and Shannon diversity indices) and bacterial richness, based on the bacterial sequence profiles.

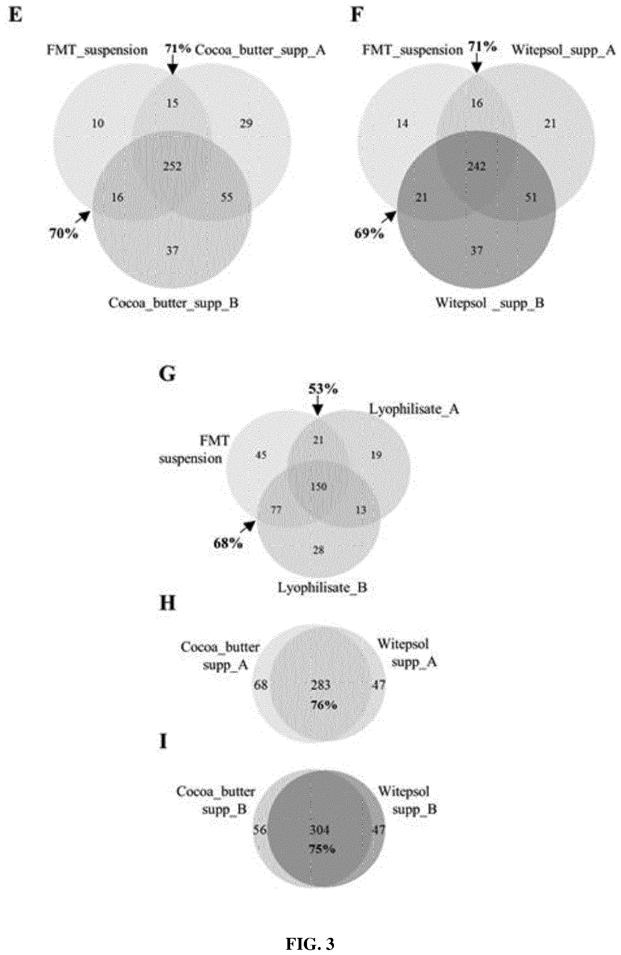

Fig. 3 **E-I** shows venn diagram of common and unique bacterial sequences between donor FMT suspension and suppositories with cocoa butter (E) and Witepsol base (F); between FMT suspension and lyophilized fecal matter with different storage conditions (G); between different storage conditions of cocoa butter and Witepsol based suppositories (H, I).

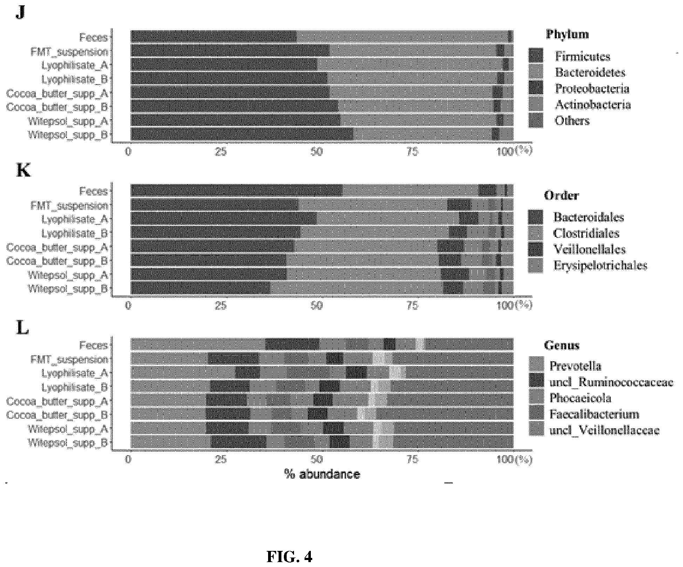

Fig. 4 **J-L** **shows** relative abundance of the top 4 phyla, top 7 order, and top 10 genus. A - storage +4°C, B - storage -80°C, Supp - suppositories.

### DETAILED DESCRIPTION OF THE INVENTION

The present invention relates to a rectal suppository, containing lyophilized donor fecal matter, that is intended to be used rectally for the purposes of treatment or prophylaxis of various gastrointestinal diseases or pathological conditions.

### Preparation of the donor fecal matter

The donor fecal matter is produced from stool that is donated by healthy volunteers. Before becoming donors, the volunteers have to sign an informed consent form and undergo the selection process. The process starts from a medical interview, which aims to assess the donor's risk of infections and past interventions that might have altered the gut microbiota. After that, blood and stool tests for potentially transmissible infectious diseases are performed. After passing the selection process, the volunteer may start donating stool, which is processed according to a standardized protocol, frozen in -80°C and stored until use. The entire donor selection and testing process, the processing and banking of the stool samples is performed according to international recommendations (3).

### Lyophilization of the donor fecal matter

Lyophilization was performed using a research freeze-dryer. The condenser is turned on -30 min before lyophilization to reach the temperature of -53°C. The solution containing the donor fecal matter vas frozen at -50°C 24 hours before the procedure. Lyophilization takes 48 hours to complete and the resulting mass is pulverized into brown-colored powder.

### Preparation of the suppositories

The suppositories are prepared using the casting method. The cast holds a mass of 2 g. Ground cocoa butter is melted down in a porcelain plate over a warm water basin (34 - 37°C). If Witepsol is used, it is melted down in the porcelain plate at 65°C and cooled to 50°C before the lyophilized fecal matter is mixed in. Warm mortar and pestle are used to grind the lyophilized donor fecal matter into fine powder and the base material introduced. The resulting mass is constantly mixed when it cools to avoid separation of base element and lyophilized donor fecal matter. Afterwards, the material is poured into suppository casts and left in -20°C temperature for 3 - 5 min until the suppositories fully form. After that they are taken out and packaged. A single suppository contains from 0,02 to 0,3 g of lyophilized donor fecal matter and 1 to 4 g base element (cocoa butter, Witepsol or other).

When the suppository enters the rectum, the base material (cocoa butter, Witepsol or other) melts and the lyophilized donor fecal matter, containing the microbiota is released.

### Examination of bacterial composition of the rectal suppositories

To confirm the presence of bacteria inside the suppositories and its similarity with donor's feces and FMT suspension we performed a bacterial 16S rRNA gene analysis. To check the presence of the same bacterial composition as in FMT suspension after lyophilization, the lyophilized material stored at different temperatures (+4°C and -80°C) was also included in the analysis. To make sure that the bacterial composition of the suppositories does not depend on the chosen base material and storage condition, suppositories with a different base (cocoa butter and Witepsol) which at all stages of production were stored at different temperatures (+4°C and -80°C) were compared with each other.

DNA was extracted from all examined samples using QIAamp Fast DNA Stool Mini Kit (Qiagen) according to the manufacturer's instructions. For the extraction we used 180 - 220 mg fecal matter, 800 µl of FMT suspension, 100 mg of lyophilized fecal matter or 420 mg pieces cut from a suppository. DNA concentrations are presented in Table 1.

The 16S rRNA gene library generation and sequencing was performed as previously described (4). In short, specific primer pair set 27F (AGAGTTTGATCCTGGCTCAG) and 338R (TGCTGCCTCCCGTAGGAGT) and dual-indexing was used in the process of PCR (cycling conditions: (98 °C, 30 s; 30 × [98 °C, 9 s; 55 °C, 60 s; 72 °C, 90 s]; 72 °C, 10 min; 10 °C, infinity). The resulting PCR products were purified and normalized utilizing the Invitrogen SequalPrep Normalization Plate Kit (Thermo Fisher Scientific, Waltham, USA). 16S rRNA gene sequencing was performed on Illumina MiSeq platform according to manufacturer's instructions with MiSeq Reagent Kit v3.

**Table 1. Nucleic acid concentration and purity per sample.**

| *Sample* | *Nucleic acid concentration* | *260*/*280* | *260*/*230* |
|---|---|---|---|
| *Unaltered fecal matter* | 90,5 ng/ul | 1,28 | 0,38 |
| *FMT suspension* | 27,1 ng/ul | 1,68 | 0,84 |
| *Lyophilized donor fecal matter* | 87,3 ng/ul | 1,63 | 0,88 |
| *Cocoa butter suppository* | 18,1 ng/ul | 1,40 | 0,66 |
| *Witepsol suppository* | 69,7 ng/ul | 1,46 | 0,67 |

Bioinformatic processing was performed as previously described (5). FastQ files were merged with Ribosomal Database Project (RDP) assembler using dada2 package (6) version 1.10.1, in R. All samples were resampled to equal the smallest library size of 9153 reads using the phyloseq package (7) returning 748 phylotypes (bacterial sequences). Phylotypes were annotated to a taxonomic affiliation based on the naive Bayesian classification (8) with a pseudo-bootstrap threshold of 80%. Relative abundances (expressed as percentages) of different microbial communities' phylogenetic ranks (phylum, order, genus) were used to examinate bacterial composition of studied samples. Bacterial richness and alpha-diversity indices (Shannon, Simpson) were calculated using the vegan package (9) from R. The data matrices comprising the percentage of abundances of each of the above-mentioned taxa were used to construct sample-similarity matrices using the Euclidean distance, where samples were ordinated by multidimensional scaling (MDS) using R.

### Results

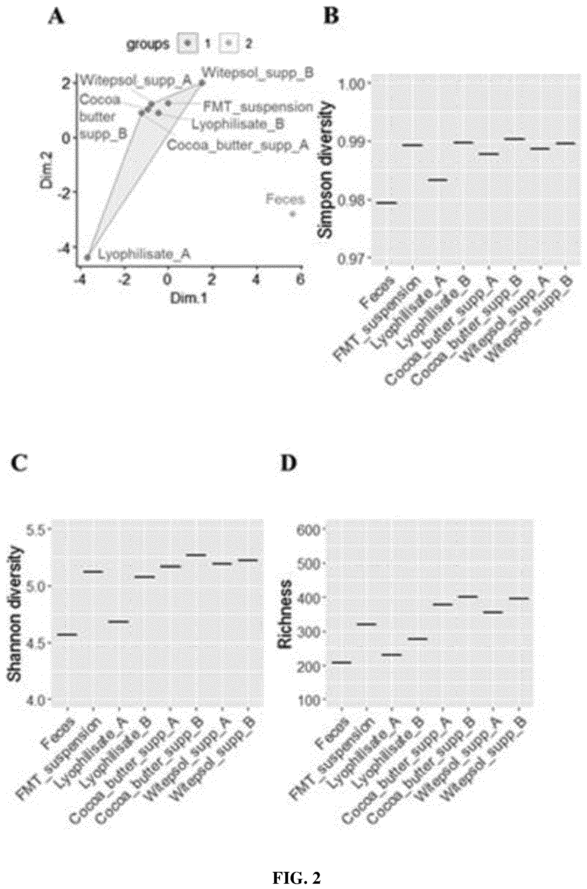

According to MDS analysis all the samples clustered in one group, except for donor's fecal sample, which was located furthest from the donor's FMT suspension, lyophilized donor fecal matter and suppositories (Fig.2 A). The bacterial alpha diversity (Simpson and Shannon diversity indices) as well as richness of the FMT suspension, lyophilized donor fecal matter stored in -80°C and both suppositories were similar (Fig.2 B-D). Even though the lyophilized donor fecal matter, which was stored at +4°C clustered in MDS analysis with FMT suspension and suppositories, its bacterial richness and alpha diversity indices were much lower. The lyophilized donor fecal matter stored at -80°C showed more common bacterial sequences with FMT suspension, compared to lyophilized donor fecal matter stored at +4°C (Fig. 2 G).

Donor's fecal sample showed lower bacterial alpha diversity and richness. The difference between the donor fecal sample and the FMT suspension, as well as fecal sample's lower richness and alpha diversity values were expected, since the feces contains solid particles from undigested food and other components, which at the same concentration of isolated DNA proportionally contain less bacterial sequences. In the current clinical practice FMT suspension of the donor is used for microbiota transplantation via endoscopy or oral capsules, therefore further comparisons were made between suppositories and the FMT suspension.

Regardless to the chosen base for the suppositories (cocoa butter or Witepsol), as well as storage conditions (+4°C or -80°C), the suppositories shared about 70% of the total bacterial sequences (Fig.2 E, F). It is noteworthy that these sequences were the most widespread, and the total abundance of unique sequences in each of the samples did not exceed more than 8.2% (Table 2).

**Table 2. Summary table of the abundance of unique bacterial sequences between FMT suspension and suppositories.**

| | *Unique bacterial sequences* | | | | | |
|---|---|---|---|---|---|---|
| | FMT suspension | | | Suppositories | | |
| | n | max abundance (%) | Sum (%) | n | max abundance (%) | Sum (%) |
| *FMT_suspension-Cocoa butter supp_A* | 26 | 0.35 | 2.7 | 84 | 0.33 | 5.8 |
| *FMT_suspension-Cocoa butter supp_B* | 25 | 0.16 | 2.1 | 92 | 0.56 | 7.9 |
| *FMT_suspension-Witepsol supp_A* | 35 | 0.26 | 3.4 | 72 | 0.32 | 6.6 |
| *FMT_suspension-Witepsol supp_B* | 30 | 0.35 | 3 | 88 | 0.46 | 8.1 |
| Supp_A - suppositories stored at +4°C. Supp_B - suppositories stored at -80°C. | | | | | | |

Suppositories with different bases also shared the main part of the detected bacterial sequences regardless of whether the lyophilized donor fecal matter and suppositories were stored at +4°C or at -80°C (Fig. 2 H, I); unique sequences abundance did not account for more than 5% in each sample as well (Table 3). The bacterial composition studied at different taxonomic levels (phylum, order, genus) of FMT suspension, lyophilized donor fecal matter stored at -80°C and all suppositories samples were very similar in percentage amounts (Fig.2 I-K).

**Table 3. Summary table of the abundance of unique bacterial sequences between suppositories with different base.**

| | *Unique bacterial sequences* | | | | | |
|---|---|---|---|---|---|---|
| | Cocoa butter suppositories | | | Witepsol suppositories | | |
| | n | max abundance (%) | Sum (%) | n | max abundance (%) | Su m (%) |
| *Cocoa butter supp_A-Witepsol supp_A* | 68 | 0.26 | 4.9 | 47 | 0.37 | 4.7 |
| *Cocoa butter supp_B-Witepsol supp_B* | 56 | 0.27 | 3.6 | 47 | 0.53 | 4.1 |
| Supp_A - suppositories stored at +4°C. Supp_B - suppositories stored at -80°C. | | | | | | |

The suppository composition is intended for use in the treatment of Clostridioides difficile colitis, antibiotic associated diarrhea, irritable bowel syndrome, functional constipation and inflammatory bowel diseases including ulcerative colitis and Crohn's disease.

### REFERENCES

1. Youngster I, Mahabamunuge J, Systrom HK, Sauk J, Khalili H, Levin J, et al. Oral, frozen fecal microbiota transplant (FMT) capsules for recurrent Clostridium difficile infection. BMC Med [Internet]. 2016;14(1):4-7. Available from: http://dx.doi.org/10.1186/s12916-016-0680-9
2. Stapleton AE, Au-Yeung M, Hooton TM, Fredricks DN, Roberts PL, Czaja CA, et al. Randomized, placebo-controlled phase 2 trial of a lactobacillus crispatus probiotic given intravaginally for prevention of recurrent urinary tract infection. Clin Infect Dis. 2011;52(10):1212-7.
3. Cammarota G, Ianiro G, Kelly CR, Mullish BH, Allegretti JR, Kassam Z, et al. International consensus conference on stool banking for faecal microbiota transplantation in clinical practice. Gut. 2019;68(12):2111-21.
4. Wang J, Thingholm LB, Skiecevičie J, Rausch P, Kummen M, Hov JR, et al. Genomewide association analysis identifies variation in Vitamin D receptor and other host factors influencing the gut microbiota. Nat Genet. 2016;48(11):1396-406.
5. Camarinha-Silva A, Jauregui R, Chaves-Moreno D, Oxley APA, Schaumburg F, Becker K, et al. Comparing the anterior nare bacterial community of two discrete human populations using Illumina amplicon sequencing. Environ Microbiol. 2014;16(9):2939-52.
6. Callahan BJ, Mcmurdie PJ, Rosen MJ, Han AW, Johnson AJA, Holmes SP. DADA2 : High-resolution sample inference from Illumina amplicon data. 2016;13(7).
7. Mcmurdie PJ, Holmes S. phyloseq : An R Package for Reproducible Interactive Analysis and Graphics of Microbiome Census Data. 2013;8(4).
8. Wang Q, Garrity GM, Tiedje JM, Cole JR, Al WET. Nai ¨ ve Bayesian Classifier for Rapid Assignment of rRNA Sequences into the New Bacterial Taxonomy. 2007;73(16):5261-7.
9. Oksanen AJ, Blanchet FG, Friendly M, Kindt R, Legendre P, Mcglinn D, et al. Vegan : Community Ecology Package Package ' vegan .' 2015; (January).

Organization Applicant
---------------------

<110> OrganizationName : Lietuvos sveikatos moksl universitetas

Application Project
-------------------
<120> Title : MODIFICATION OF FECAL MICROBIOTA WITH RECTAL SUPPOSITORIES
<130> AppFileReference : EPP2021/27
<140> CurrentAppNumber : EP21162346.7
<141> CurrentFilingDate : 2021-03-12

Sequence
<212> Type: DNA
<211> Lenght: 19
--------
<213> OrganismName : bacteria
<221> FeatureKey: primer
<400> SEQ ID NO:1

tgctgcctcc cgtaggagt                                                    19

<212> Type: DNA
<211> Lenght: 19
--------
<213> OrganismName : bacteria
<221> FeatureKey: primer
<400> SEQ ID NO:2

tgctgcctcc cgtaggagt                                                    19

Organization Applicant
----------------------

<110> OrganizationName : Lietuvos sveikatos moksl universitetas

Application Project
-------------------
<120> Title : MODIFICATION OF FECAL MICROBIOTA WITH RECTAL SUPPOSITORIES
<130> AppFileReference : EPP2021/27
<140> CurrentAppNumber : EP21162346.7
<141> CurrentFilingDate : 2021-03-12

Sequence
<212> Type: DNA
<211> Lenght: 20
--------
<213> OrganismName : bacteria
<221> FeatureKey: primer
<400> SEQ ID NO:1

agagtttgat cctggctcag                                                    20

<212> Type: DNA
<211> Lenght: 19
--------
<213> OrganismName : bacteria
<221> FeatureKey: primer
<400> SEQ ID NO:2

tgctgcctcc cgtaggagt                                                     19

## Claims

1. Suppository composition for rectal administration, consisting of lyophilized donor fecal matter and base component.

2. Suppository composition according to claim 1, wherein the composition comprises donor fecal microbiota.

3. Suppository composition according to claim 1 or 2, wherein base component is cocoa butter.

4. Suppository composition according to claim 1 or 2, wherein base component is Witepsol.

5. Suppository composition according to any of claims 1-4, wherein the composition is formulated for rectal administration.

6. A method for preparing a suppository composition according to claims 1-5, wherein said method comprises:
a) melting of base component;
b) grinding fecal matter into fine powder;
c) mixing fecal matter with cocoa butter;
d) forming suppositories and left until suppositories fully form.

7. A method according to claim 6, wherein basic component of step a) is cocoa butter melted at 34-37°C.

8. A method according to claim 6, wherein basic component of step a) is Witepsol melted at 60-70°C.

9. A method according to claim 8, wherein the Witepsol in a) step is cooled to 47-53°C before the mixing with fecal matter.

10. A method according to any of claims 6 - 9, wherein donor fecal matter is lyophilized before step b).

11. A method according to any of claims 6 - 10, wherein resulting mass is constantly mixed when it cools to avoid separation of base element and lyophilized donor fecal matter.

12. A suppository composition, for use in the treatment of disease or pathological condition of the colon.

13. A suppository composition, for use according to claim 12, wherein the disease or pathological condition is selected from *Clostridioides difficile* colitis, antibiotic associated diarrhea, irritable bowel syndrome, functional constipation and inflammatory bowel diseases.

14. A suppository composition, for use according to any of claim 12 and 13, wherein inflammatory bowel disease are ulcerative colitis and Crohn's disease.
